Europäisches Patentamt

European Patent Office

Office européen des brevets

(11) Veröffentlichungsnummer: **0 077 527**
**A2**

(12) ## EUROPÄISCHE PATENTANMELDUNG

(21) Anmeldenummer: **82109480.2**

(22) Anmeldetag: **13.10.82**

(51) Int. Cl.³: **A 61 B 6/00**

(30) Priorität: **19.10.81 US 312621**
**18.02.82 US 349761**

(43) Veröffentlichungstag der Anmeldung:
**27.04.83 Patentblatt 83/17**

(84) Benannte Vertragsstaaten:
**DE FR**

(71) Anmelder: **SIEMENS AKTIENGESELLSCHAFT**
**Berlin und München Wittelsbacherplatz 2**
**D-8000 München 2(DE)**

(72) Erfinder: **Platz, Winfried**
**50 Roaring Brook**
**Southington Connecticut 06489(US)**

(72) Erfinder: **Plummer, Steven J.**
**12 Burt Drive**
**Middlefield Connecticut 06455(US)**

(72) Erfinder: **Wieloch, James E.**
**872 South End Road**
**Southington Connecticut 06489(US)**

(54) Röntgendiagnostikeinrichtung.

(57) Die Erfindung betrifft eine Röntgendiagnostikeinrichtung mit einer Röntgenröhre (10), mit Mitteln zur Bewegung der Röntgenröhre (10) zwischen wenigstens einer ersten Stellung, in der die Röntgenröhre (10) ein Röntgenstrahlenbündel (18) in einer ersten Richtung aussendet, und einer zweiten Stellung, in der die Röntgenröhre (10) ein Röntgenstrahlenbündel (18) in einer zweiten Richtung senkrecht zu der ersten Richtung aussendet, und mit zwei Filmkassettenhaltern (8, 20) für die Belichtungen in beiden Stellungen der Röntgenröhre (10). Die Röntgenröhre (10) und mindestens ein Filmkassettenhalter (20) sind durch Bewegungsmittel (24) verbunden, die den Filmkassettenhalter (20) in einer Warteposition außerhalb der Belichtungsposition halten, wenn die Röntgenröhre (10) sich in ihrer ersten Stellung befindet, und den Filmkassettenhalter (20) von der Warteposition in die Belichtungsposition überführen, wenn die Röntgenröhre (10) von ihrer ersten Stellung in ihre zweite Stellung bewegt wird.

FIG 1

**0077527**

SIEMENS AKTIENGESELLSCHAFT           Unser Zeichen
Berlin und München                   VPA 81 P 8250 kb E

<u>Röntgendiagnostikeinrichtung</u>

Die Erfindung betrifft eine Röntgendiagnostikeinrichtung mit einer Röntgenröhre, mit Mitteln zur Bewegung
der Röntgenröhre zwischen wenigstens einer ersten Stellung, in der die Röntgenröhre ein Röntgenstrahlenbündel
in einer ersten Richtung aussendet, und einer zweiten
Stellung, in der die Röntgenröhre ein Röntgenstrahlenbündel in einer zweiten Richtung senkrecht zu der ersten
Richtung aussendet, und mit zwei Filmkassettenhaltern
für die Belichtung in beiden Stellungen der Röntgenröhre. Derartige Einrichtungen ermöglichen Aufnahmen
in vertikalen und horizontalen Richtungen eines Patienten.

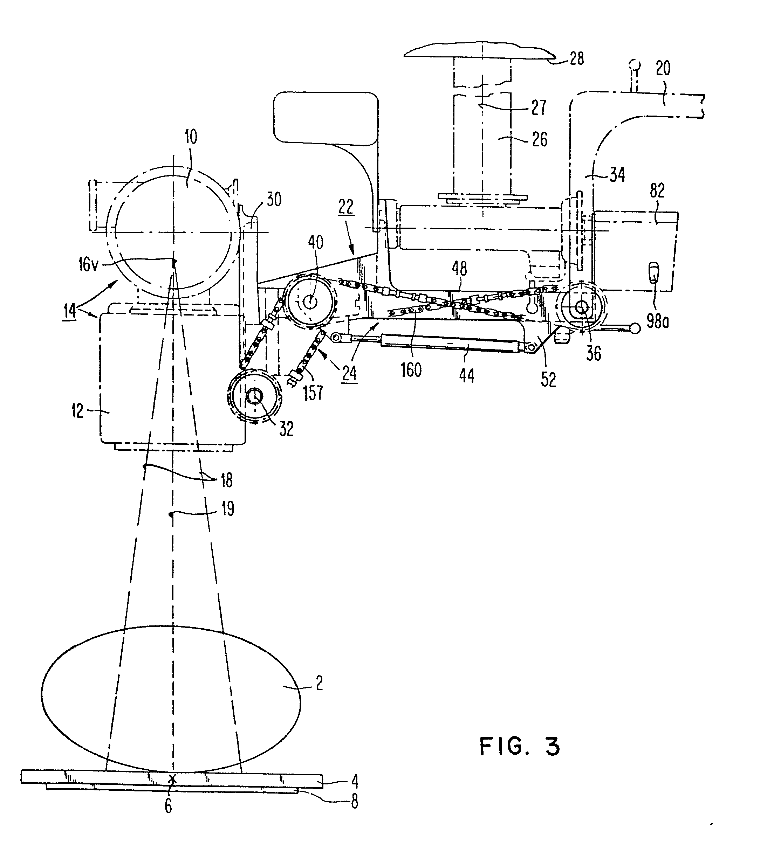

In dem Datenblatt "Deckenstative 3 D und 3 D - M zum
Anbau von Röntgenstrahlern und Bildverstärkern" der
Firma SIEMENS AG ist ein Deckenstativ beschrieben, das
in der Röntgendiagnostik als Träger für einen Röntgenstrahler oder einen Bildverstärker vorgesehen ist. Das
Deckenstativ besteht aus einer vertikalen Teleskopsäule, die an der Decke angebracht ist. Die Teleskopsäule kann in longitudinalen und transversalen Laufbahnen bewegt werden oder auch fest an der Decke montiert sein, so daß nur vertikale Bewegungen möglich
sein. In Figur 3 des obengenannten Datenblattes ist
an dem unteren Ende der Teleskopsäule ein horizontal
ausgerichteter Tragarm angebracht. Der Röntgenstrahler,
der aus einer Röntgenröhre mit Gehäuse und einem Kollimator besteht, ist an dem einen Ende des Tragarmes angeordnet, an dessen anderen Ende ein Bedienungskasten

Gse 5 Rl / 04.10.1982

angebracht ist, der Steuerschalter, eine Winkelanzeige für die Schrägstellung des Röntgenstrahlers, ein Lichtvisier zum Zentrieren des Röntgenstrahlers auf den Kassettenhalter, der in einem unterhalb des Deckenstativs angeordneten Bucky-Tisch angeordnet ist, und ein Meßband zur Einstellung des Fokus-Film-Abstandes bei Schräg- und Horizontalstrahlung enthält. Aufnahmen mit vertikaler Strahlung sind ebenfalls möglich. Die Schalter in dem Bedienungskasten arretieren den Röntgenstrahler.

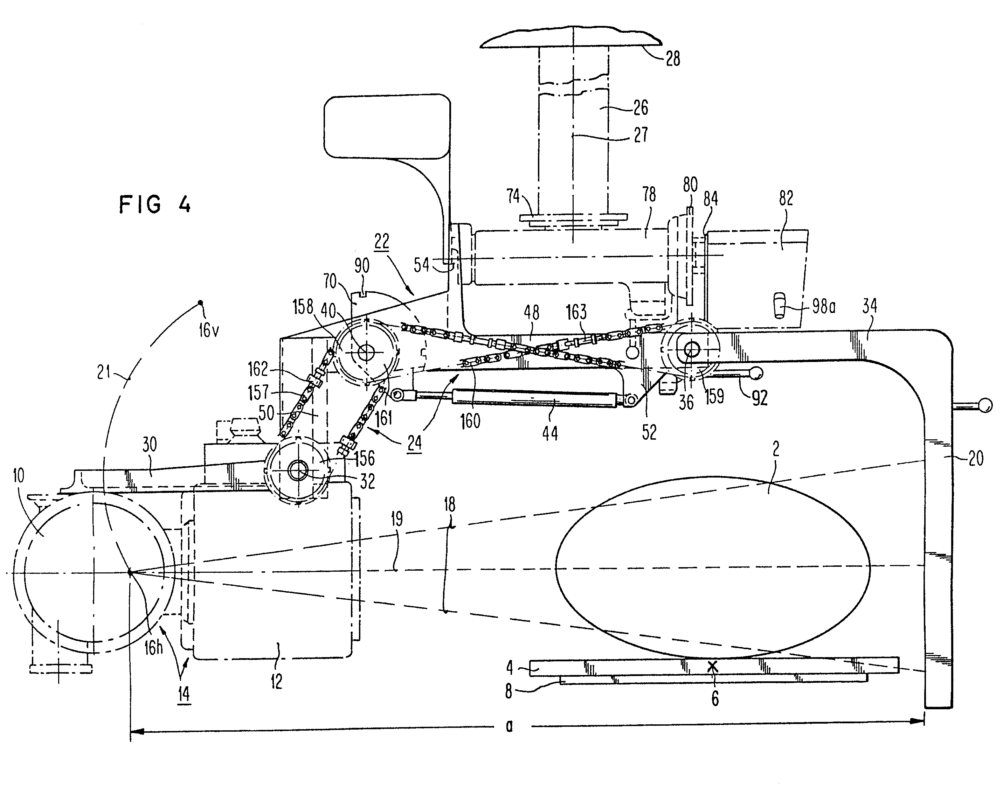

Das Deckenstativ ermöglicht wahlweise Röntgenbelichtungen eines Patienten in vertikaler und horizontaler Richtung. Ein erster Filmkassettenhalter ist in dem Tischoberteil unterhalb des Patienten angebracht. Für horizontale Belichtungen wird ein zweiter Filmkassettenhalter benutzt, der in einem Bucky-Wandgerät (s. Figur 4 des Datenblattes) angeordnet ist.

Um die Röntgenröhre von ihrer vertikalen Belichtungsposition in ihre horizontale Belichtungsposition zu bewegen, muß der Röntgenstrahler um die Longitudinalachse des Tragarmes gedreht werden. Dieses Deckenstativ ermöglicht also Belichtungen in vertikaler Richtung unter Benutzung des in dem Bucky-Tisch eingebauten Filmkassettenhalters und horizontale Belichtungen einer zweiten Filmkassette, die in einem zusätzlichen Wandgerät eingebaut ist. Dieses zusätzliche Wandgerät für die horizontale Belichtung verteuert die Röntgendiagnostikeinrichtung. Zusätzlich erfordert das Umstellen von der vertikalen zur horizontalen Belichtungsposition Zeit, weil der Röntgenstrahler auf die zweite Kassette erneut ausgerichtet werden muß. Jedesmal muß zur Bestimmung der korrekten Belichtungszeit der Fokus-Film-Abstand gemessen werden. Da der zweite Filmkassettenhalter in dem Wandgerät angebracht ist, ist der Zugang zu dem Patienten, insbesondere
bei lateralen Belichtungen begrenzt.

In der DE-PS 21 41 461 ist ein Röntgengerät beschrieben,
das ein Deckenstativ, eine Röntgenröhre und einen Röntgenbildverstärker oder eine Filmkassette enthält. An
dem unteren Ende des Deckenstativs ist ein um eine
senkrecht zu der Longitudinalachse der Säule angeordnete
Achse drehbarer Ausleger angebracht. An den Enden dieses
Auslegers sind zwei Tragarme drehbar verbunden, an deren
freien Enden die Röntgenröhre und die Filmkassette drehbar angebracht sind. Alle drehbaren Achsen sind parallel
zueinander angeordnet. Ein Antriebsmechanismus ermöglicht unabhängig von der Drehposition des Auslegers, daß
der Röntgenstrahler und der Röntgenbildverstärker immer
aufeinander ausgerichtet sind und der Zentralstrahl
des Röntgenstrahlenbündels immer parallel zu der
Longitudinalachse des Auslegers gehalten wird. Wegen
des schwierigen Antriebsmechanismus ist die Produktion
eines derartigen Röntgengerätes teuer. Außerdem hat
die Bedienungsperson keinen freien Zugang zu dem Patienten.

Bei Untersuchungen von inneren Organen eines Patienten,
beispielsweise des Magens, wird beispielsweise die
Reaktion von Barium-Kontrastbrei auf die Magenwand
eines auf einem Untersuchungstisch liegenden Patienten
beobachtet. Zu diesem Zwecke werden Aufnahmen quer zur
Longitudinalachse des Patienten gemacht. Bei Seitenlage
des Patienten erfolgen dorsale oder ventrale Belichtungen und für laterale Belichtungen liegt der Patient
auf dem Rücken oder dem Bauch. Dabei ist das von der
Röntgenröhre ausgehende Röntgenstrahlenbündel horizontal
ausgerichtet und belichtet den vertikal angeordneten
Röntgenfilm.

Die Erfindung geht von der Aufgabe aus, eine Röntgendiagnostikeinrichtung der eingangs genannten Art zu schaffen, die einen schnellen Wechsel zwischen beiden Stellungen ermöglicht, der durch einfache und kompakte Mittel ermöglicht wird, und bei der der Zugang zu dem zu untersuchenden Patienten nicht durch den nicht benötigten Filmkassettenhalter behindert wird.

Die Aufgabe wird erfindungsgemäß dadurch gelöst, daß die Röntgenröhre und mindestens ein Filmkassettenhalter durch Bewegungsmittel verbunden sind, die den Filmkassettenhalter in einer Warteposition außerhalb der Belichtungsposition halten, wenn die Röntgenröhre sich in ihrer ersten Stellung befindet, und den Filmkassettenhalter von der Warteposition in die Belichtungsposition überführen, wenn die Röntgenröhre von ihrer ersten Stellung in die zweite Stellung bewegt wird. Durch diese Bewegungsmittel wird ein schneller Wechsel zwischen den beiden Stellungen der Röntgenröhre und des Filmkassettenhalters ermöglicht, wobei durch Drehung der einen Einheit die andere automatisch in ihre zugehörige Stellung bewegt wird.

Die Röntgendiagnostikeinrichtung weist einen einfachen Aufbau auf, wenn an der Trägersäule eine Befestigungsvorrichtung angebracht ist, wenn erste Drehmittel an der einen Seite der Befestigungsvorrichtung angeordnet sind, die ein Kippen der Röntgenröhre um eine erste horizontale Achse zwischen der ersten und der zweiten Stellung ermöglichen, und wenn zweite Drehmittel an der gegenüberliegenden Seite der Befestigungsvorrichtung angebracht sind, die ein Kippen des Filmkassettenhalters um eine zweite horizontale Achse zwischen der Warteposition und der Belichtungsposition ermöglichen. Der Zugang des Bedienungspersonals zu dem Patienten wird erleichtert, wenn der Filmkassettenhalter in seiner Warteposition sich oberhalb der Befestigungsvorrichtung befindet und die Röntgenröhre in ihrer ersten Stellung ein nach unten gerichtetes Röntgenstrahlenbündel aussendet, und wenn der Filmkassettenhalter in seiner Belichtungsposition vertikal ausgerichtet ist und die Röntgenröhre in ihrer zweiten Stellung ein horizontales Röntgenstrahlenbündel auf den Filmkassettenhalter zu aussendet.

Es hat sich als vorteilhaft erwiesen, wenn die die Röntgenröhre und den Filmkassettenhalter verbindenden Bewegungsmittel Kettentriebe oder Hebel sind.  Eine exaktere Führung wird erreicht, wenn die die Röntgenröhre und den Filmkassettenhalter verbindenden Bewegungsmittel zwei Systeme enthalten, die parallel zueinander, seitlich der Befestigungsvorrichtung angeordnet sind.

Die Erfindung wird anhand von in der Zeichnung dargestellten Ausführungsbeispielen näher erläutert. Es zeigen:

Fig. 1 eine schematische Seitenansicht einer ersten Ausführungsform einer Röntgendiagnostikeinrichtung nach der Erfindung, bei der sich die Röntgenröhre in einer vertikalen Strahlungsposition und der zweite Filmkassettenhalter in seiner Warteposition befinden,

Fig. 2 eine schematische Seitenansicht der ersten Ausführungsform gemäß Figur 1, bei der sich die Röntgenröhre in ihrer horizontalen Strahlungsposition und der zweite Filmkassettenhalter in seiner Belichtungsposition befinden,

Fig. 3 eine Seitenansicht einer zweiten Ausführungsform einer Röntgendiagnostikeinrichtung nach
der Erfindung, bei der sich die Röntgenröhre
und der zweite Filmkassettenhalter in einer
Stellung gemäß Figur 1 befinden,

Fig. 4 eine Seitenansicht der zweiten Ausführungsform gemäß Figur 2, bei der sich die Röntgenröhre und der zweite Filmkassettenhalter in
einer Stellung gemäß Figur 2 befinden und

Fig. 5 eine perspektivische Ansicht der in Figur 4
dargestellten Röntgendiagnostikeinrichtung.

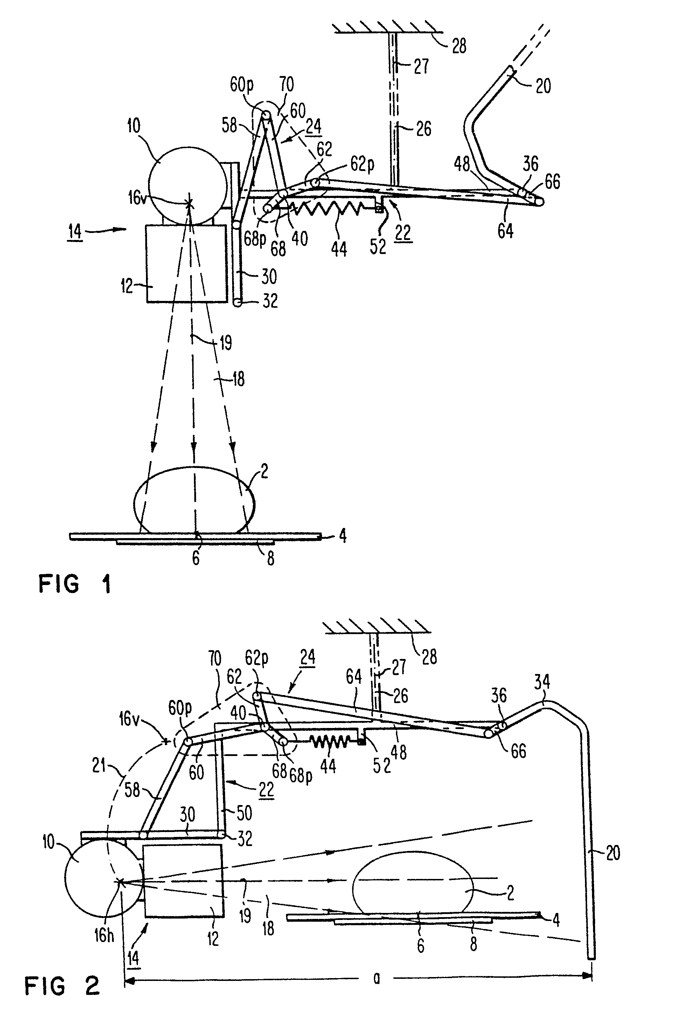

In den Figuren 1 und 2 ist eine Röntgendiagnostikeinrichtung dargestellt, die vertikale und horizontale
Aufnahmen eines Patienten 2 erstellen kann. Der Patient 2 liegt auf einem Tischoberteil 4 eines konventionellen Untersuchungstisches. Unterhalb des Tischoberteils 4 ist ein erster Filmkassettenhalter 8 angebracht.

Die Röntgendiagnostikeinrichtung weist eine Röntgenröhre 10 auf, die mit einem Kollimator 12 einen Röntgenstrahler 14 bildet. In Figur 1 nimmt die Röntgenröhre 10 eine erste Stellung ein, in welcher von ihrem
Fokus 16v ein divergierendes Röntgenstrahlenbündel 18
ausgeht, dessen Zentralstrahl 19 vertikal nach unten
auf den Patienten 2 und den ersten Filmkassettenhalter
8 ausgerichtet ist. In der Figur 2 nimmt die Röntgenröhre 10 eine zweite Stellung ein, in welcher sie von
ihrem Fokus 16h ein horizontal gerichtetes Röntgenstrahlenbündel 18 auf den Patienten 2 aussendet. Die Bewegung
des Fokus 16 zwischen den beiden Stellungen verläuft
auf einem Viertelkreis 21.

Der erste Filmkassettenhalter 8 wird für die in Figur 1 dargestellten vertikalen Belichtungen benötigt. Er weist eine horizontal ausgerichtete Fläche auf, die das vertikale Röntgenstrahlenbündel 18 der in ihrer ersten Stellung befindlichen Röntgenröhre 10  empfängt. Ein zweiter Filmkassettenhalter 20, dessen Fläche in seiner Belichtungsposition vertikal angeordnet ist, empfängt das horizontale Röntgenstrahlenbündel 18 der in ihrer zweiten Stellung befindlichen Röntgenröhre 10 (Figur 2). In Figur 1 ist der zweite Filmkassettenhalter 20 in einer Warteposition außerhalb des vertikalen Röntgenstrahlenbündels 18 dargestellt.

Zur gleichzeitigen Bewegung der Röntgenröhre 10 von ihrer ersten zu ihrer zweiten Stellung und des zweiten Filmkassettenhalters 20 von seiner Warteposition in seine Belichtungsposition sind an einer Befestigungsvorrichtung 22 Bewegungsmittel 24 beweglich angebracht. Die Befestigungsvorrichtung 22 ist an dem unteren Ende einer Trägersäule 26, beispielsweise einer Teleskopsäule, angebracht, die sich von der Decke 28 in vertikaler Richtung nach unten erstreckt. An der linken Seite der Befestigungsvorrichtung 22 ist die Röntgenröhre 10 angeordnet.

Die Röntgenröhre 10 ist an einem ersten, um eine erste horizontale Achse 32 kippbaren Hebelarm 30 angebracht. An der rechten Seite der Befestigungsvorrichtung 22 ist der zweite Filmkassettenhalter 20 mit einem gebogenen, um eine zweite horizontale Achse 36 drehbaren zweiten Hebelarm 34 verbunden. Beide Achsen 32 und 36 sind parallel zueinander  an den Endteilen der Befestigungsvorrichtung 22 angeordnet. Die erste Achse 32 liegt unterhalb der zweiten Achse 36.

Die Koordination der beiden Bewegungen wird durch die
Bewegungsmittel 24 bewirkt, die um eine gemeinsame,
dritte horizontale Achse 40 drehbar angeordnet sind.
Diese dritte horizontale Achse 40 ist parallel zu den
Achsen 32 und 36 und befindet sich in gleicher Höhe
mit der zweiten Achse 36. Die drei horizontalen Achsen
32, 36, 40 sind unterhalb der Trägersäule 26 an der
Befestigungsvorrichtung 22 angebracht.

Da der zweite Filmkassettenhalter 20 durch die Bewegungsmittel 24 mit der Röntgenröhre 10 verbunden ist,
wird der zweite Filmkassettenhalter 20 automatisch um
die zweite horizontale Achse 36 gedreht, wenn die Bedienungsperson, ein Arzt oder eine Schwester, die Röntgenröhre 10 um die erste horizontale Achse 32 dreht.
Dieser Vorgang wird durch eine Feder 44 erleichtert,
die die Gewichte des Röntgenstrahlers 14 auf der einen
Seite und des zweiten Filmkassettenhalters 20 auf der
anderen Seite ausbalanciert. Auch wenn die Bedienungsperson den zweiten Filmkassettenhalter 20 um die zweite
horizontale Achse 36 dreht, ergibt sich automatisch
eine korrespondierende Drehung der Röntgenröhre 10.

Die Befestigungsvorrichtung 22 besteht aus einem horizontal angeordneten Trägerarm 48, einem Verlängerungsarm 50, der sich vom linken Ende des Trägerarmes 48 nach
unten erstreckt, und einem schmalen Haltestück 52, das
an dem Mittelteil des Trägerarmes 48 angebracht ist. Die
erste horizontale Achse 32 ist an dem unteren Ende des
vertikalen Verlängerungsarmes 50, die zweite horizontale Achse 36 ist an dem rechten Ende des Trägerarmes
48 und die dritte horizontale Achse 40 ist zwischen dem
linken Ende des Trägerstückes 48 und der Befestigung
der Trägersäule 26 angeordnet, so daß in der horizontalen Belichtungsposition (Figur 2) die Röntgenröhre

10 und der zweite Filmkassettenhalter 20 sich unterhalb der Trägersäule 26 befinden.

Die Bewegungsmittel 24 enthalten für die Röntgenröhre 10 ein erstes Kniegelenk, das aus zwei drehbaren Hebelarmen 58 und 60 gebildet wird. Das äußere Ende des Hebelarmes 58 ist mit dem mittleren Teil des ersten Hebelarmes 30 und das äußere Ende des Hebelarmes 60 mit der dritten horizontalen Achse 40 drehbar verbunden. Die Bewegungsmittel 24 für den Filmkassettenhalter 20 weisen ein zweites Kniegelenk auf, das zwei weitere Hebelarme 62 und 64 umfaßt. Das äußere Ende des Hebelarms 62 ist mit der dritten horizontalen Achse 40 und das äußere Ende des Hebelarms 64 mit einer Verlängerung 66 des gebogenen Hebelarmes 34 über die zweite horizontale Achse 36 hinaus drehbar verbunden. Ein weiterer Hebelarm 68 ist ebenfalls mit der dritten horizontalen Achse 40 drehbar verbunden, an dessen äußeren Ende die Feder 44, beispielsweise eine Gasdruckfeder, befestigt ist. Mit ihrer anderen Seite ist die Feder 44 an dem Haltestück 52 angebracht. Die Hebelarme 60, 62 und 68 sind starr miteinander verbunden und bilden einen Stern. Sie sind gemeinsam um die dritte horizontale Achse 40 drehbar.

Wie in den Figuren 1 und 2 gestrichelt dargestellt, können die Hebelarme 60, 62 und 68 in einer Platte 70 vereinigt werden, die drei Rotationsachsen 60p, 62p und 68p aufweist, die den Drehachsen der beiden Kniegelenke und dem Angriffspunkt der Feder 44 entsprechen. Die Platte 70 dreht sich um die an der Befestigungsvorrichtung 22 angebrachte dritte horizontale Achse 40.

Zur Justierung der Röntgenröhre 10 und des zweiten Filmkassettenhalters 20 können an den Armen 58 und/ oder 64 Zwischenstücke angebracht sein.

In den Figuren 3 und 4 ist eine zweite Ausführungsform der Röntgendiagnostikeinrichtung dargestellt, bei der die Koppelung der beiden Bewegungen durch Kettentriebe erfolgt. Der übrige Aufbau entspricht auch im Hinblick auf die Bezugszeichen der Ausführung der Figuren 1 und 2.

Die Montageeinrichtung 22 besteht aus dem horizontal angeordneten Trägerarm 48, dem Verlängerungsarm 50, dem Haltestück 52 und einem Verbindungsstück 54. Die Teile 48, 50, 52 und 54 können als ein einstückiges Gußteil hergestellt sein, daß die drei parallelen horizontalen Achsen 32, 36 und 40 enthält, die nunmehr Rotationsachsen für Kettenräder sind.

Die Bewegungsmittel 24 weisen auf der Seite der Röntgenröhre 10 ein erstes Kettenrad 156, eine Kette 157 und ein zweites Kettenrad 158 auf. Das erste Kettenrad 156 ist fest mit der Außenseite des ersten Hebelarmes 30 verbunden und mit diesem um die erste horizontale Achse 32 drehbar. Das zweite Kettenrad 158 ist an der dritten Achse 40 drehbar angeordnet. Die Kette 157 verbindet die Kettenräder 156 und 158.

Auf der Seite des Filmkassettenhalters 20 weisen die Bewegungsmittel 24 ein drittes Kettenrad 159, eine Kette 160 und ein viertes Kettenrad 161 auf. Das dritte Kettenrad 159 ist fest an der Innenseite des zweiten Hebelarmes 34 angebracht und mit diesem um die zweite horizontale Achse 36 drehbar. Das vierte Kettenrad 161 ist fest mit dem zweiten Kettenrad 158 verbunden. Die Kette 160 verbindet über Kreuz die Kettenräder 159 und 161 miteinander. Diese Kettenanordnung bewirkt eine Umkehr der Bewegung, so daß eine gleichzeitige Ab- und Aufwärtsbewegung des Filmkassettenhalters 20 und des Röntgenstrahlers 14 ermöglicht wird.

In die Ketten 157 und 160 sind Spannschlösser 162 und 163 eingefügt, die unabhängig voneinander die Justierung des Röntgenstrahlers 14 und des Filmkassettenhalters 20 erlauben.

Durch die Befestigungsvorrichtung 22 und die Bewegungsmittel 24 kann ein schneller Wechsel von einer vertikalen Belichtungsposition (Figuren 1 und 3) in eine horizontale Belichtungsposition (Figuren 2 und 4) erreicht werden. Die Feder 44 erleichtert diesen Wechsel, so daß der von der Bedienungsperson (Arzt, Schwester) aufzubringende Kraftaufwand gering bleibt.

Durch die mit kleinem Spiel hergestellten mechanischen Teile kann der Film-Fokus-Abstand a in der horizontalen Belichtungsposition auch nach einer großen Anzahl von Wechseln zwischen der ersten und der zweiten Position eingehalten werden. Der Abstand a zwischen dem Fokus 16h und dem zweiten Filmkassettenhalter 20 kann beispielsweise 1 m betragen.

An dem unteren Ende der Trägersäule 26 ist ein Drehtisch 74 zur Drehung der Befestigungsvorrichtung 22 um die longitudinale Achse 27 der Trägersäule 26 angebracht. Dadurch kann die gesamte Röntgenanordnung um die vertikale Achse 27 gedreht werden.

An der unteren Seite des Drehtisches 74 ist ein horizontal angeordneter Tragarm 78 angebracht, an dessen rechter Seite eine Drehvorrichtung 80 angebracht ist.

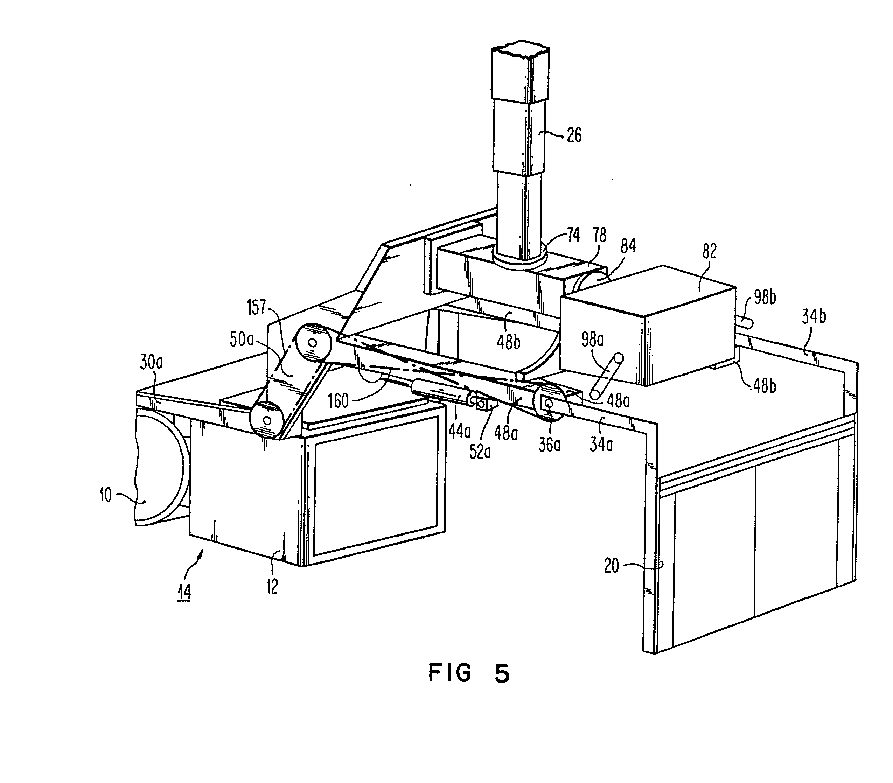

Die Drehvorrichtung 80 trägt einen Bedienungskasten 82, der Steuerelemente für die Bedienung der Röntgendiagnostikeinrichtung aufnimmt, so daß er um eine horizontale Achse gedreht werden kann. Der Bedienungskasten 82 kann, wie in der Figur 5 dargestellt, mit Handgriffen 98a und 98b versehen sein, die diese Drehbewegung ermöglichen. Das linke Ende des Tragarms 78 ist mit dem Verbindungsstück 54 der Befestigungsvorrichtung 22 fest verbunden.

Bei der in dem Stand der Technik beschriebenen Röntgendiagnostikeinrichtung ist der Röntgenstrahler 14 direkt mit dem Tragarm 78 verbunden. Eine nachträgliche Umrüstung dieser Röntgendiagnostikeinrichtung kann leicht durch das Abnehmen des Röntgenstrahlers 14 von dem Tragarm 78 erreicht werden. An dessen Stelle wird die Befestigungsvorrichtung 22 mit dem Verbindungsstück 54 und dem vorderen Befestigungsträger 84 an dem Tragarm 78 angebracht. An dem Hebelarm 30 der Befestigungsvorrichtung 22 wird dann der Röntgenstrahler 14 befestigt.

In der Figur 5 ist eine perspektivische Ansicht der Röntgendiagnostikeinrichtung gemäß Figuren 3 und 4 dargestellt. Die Elemente, welche an der Vorderseite (entsprechend den Figuren 1 und 2) des Röntgengerätes dargestellt sind, sind mit einem Bezugszeichen a und die an der Rückseite befestigten Teile mit b bezeichnet.

Aus Figur 5 läßt sich ersehen, daß zwei parallele Mechanismen für einen koordinierten Wechsel der Röntgenröhrenanordnung 14 und des zweiten Filmkassettenhalters 20 gebraucht werden. Der Röntgenstrahler 14 wird durch die parallelen Hebelarme 30a und 30b getragen, während der zweite Filmkassettenhalter 20 durch die parallelen Hebelarme 34a und 34b gehalten wird. Der Hebelarm 30a ist funktionsmäßig mit dem Hebelarm 34a durch einen in den Figuren 3 und 4 dargestellten Kettentrieb verbunden. Der Hebelarm 30b ist mit dem Arm 34b durch einen Kettentrieb funktionell verbunden, der zu dem in den Figuren 3 und 4 dargestellten invertiert aufgebaut ist.

Die Röntgendiagnostikeinrichtung weist eine nicht dargestellte Sperrvorrichtung auf, die unbeabsichtigte Bewegungen des Röntgenstrahlers 14 und des zweiten Filmkassettenhalters 20 verhindert. Diese Sperrvorrichtung besteht aus einer Stange, die in einen von zwei um 90° versetzte, an dem Rande der Platte 70 angeordnete Schlitze 90 entsprechend der gewünschten Belichtungsposition eingreift. Die Stange wird durch einen Bedienungshebel 92 aus einem Schlitz 90 ausgerückt und gibt die Platte 70 frei. Durch eine Feder rastet die Stange automatisch wieder ein.

8 Patentansprüche
5 Figuren

Patentansprüche

1. Röntgendiagnostikeinrichtung mit einer Röntgenröhre (10), mit Mitteln zur Bewegung der Röntgenröhre (10) zwischen

wenigstens einer ersten Stellung, in der die Röntgenröhre (10) ein Röntgenstrahlenbündel (18) in einer ersten Richtung aussendet, und

einer zweiten Stellung, in der die Röntgenröhre (10) ein Röntgenstrahlenbündel (18) in einer zweiten Richtung senkrecht zu der ersten Richtung aussendet, und

mit zwei Filmkassettenhaltern (8, 20) für die Belichtungen in beiden Stellungen der Röntgenröhre (10),

d a d u r c h   g e k e n n z e i c h n e t ,   daß die Röntgenröhre (10) und mindestens ein Filmkassettenhalter (20) durch Bewegungsmittel (24) verbunden sind, die den Filmkassettenhalter (20) in einer Warteposition außerhalb der Belichtungsposition halten, wenn die Röntgenröhre (10) sich in ihrer ersten Stellung befindet, und den Filmkassettenhalter (20) von der Warteposition in die Belichtungsposition überführen, wenn die Röntgenröhre (10) von ihrer ersten Stellung in ihre zweite Stellung bewegt wird.

2. Röntgendiagnostikeinrichtung nach Anspruch 1, mit einer Trägersäule (26) für die Röntgenröhre (10), d a d u r c h   g e k e n n z e i c h n e t ,

daß an der Trägersäule (26) eine Befestigungsvorrichtung (22) angebracht ist,

daß erste Drehmittel an der einen Seite der Befestigungsvorrichtung (22) angeordnet sind, die
ein Kippen der Röntgenröhre (10) um eine erste
horizontale Achse (32) zwischen der ersten und
der zweiten Stellung ermöglichen, und

daß zweite Drehmittel an der gegenüberliegenden
Seite der Befestigungsvorrichtung (22) angebracht
sind, die ein Kippen des Filmkassettenhalters (20)
um eine zweite horizontale Achse (36) zwischen der
Warteposition und der Belichtungsposition ermöglichen.

3. Röntgendiagnostikeinrichtung nach Anspruch 2,    d a -
d u r c h   g e k e n n z e i c h n e t ,    daß der
Filmkassettenhalter (20) in seiner Warteposition sich
oberhalb der Befestigungsvorrichtung (22) befindet und
die Röntgenröhre (10) in ihrer ersten Stellung ein nach
unten gerichtetes Röntgenstrahlenbündel (18) aussendet,
und daß der Filmkassettenhalter (20) in seiner Belichtungsposition vertikal ausgerichtet ist und die Röntgenröhre (10) in ihrer zweiten Stellung ein horizontales
Röntgenstrahlenbündel (18) auf den Filmkassettenhalter
zu aussendet.

4. Röntgendiagnostikeinrichtung nach Anspruch 2 oder 3,
d a d u r c h   g e k e n n z e i c h n e t ,    daß
die Röntgenröhre (10) an wenigstens einem ersten Hebelarm (30) und der Kassettenhalter (20) an wenigstens
einem Hebelarm (34) befestigt sind, die um zwei parallele
horizontale Achsen (32, 36) schwenkbar sind, und daß der
Kassettenhalter (20) mit zwei gebogenen Hebelarmen (34a,
34b) verbunden ist, die parallel zueinander angeordnet
sind.

5. Röntgendiagnostikeinrichtung nach einem der Ansprüche 1 bis 4, d a d u r c h   g e k e n n z e i c h n e t , daß die die Röntgenröhre (10) und den Filmkassettenhalter (20) verbindenden Bewegungsmittel (24) Kettentriebe sind, die ein erstes, um eine erste horizontale Achse (32) drehbares Kettenrad (156), ein zweites, um eine gemeinsame horizontale Achse (40) drehbares Kettenrad (158), ein drittes, um eine zweite horizontale Achse (36) drehbares Kettenrad (159), ein viertes, ebenfalls um die gemeinsame horizontale Achse (40) drehbares Kettenrad (161), eine erste Kette (157), die das erste und zweite Kettenrad (156, 158) miteinander verbindet, und eine zweite Kette (160) aufweisen, die das dritte und vierte Kettenrad (159, 161) miteinander verbindet, wobei eine der Ketten (157 oder 160) die Kettenräder (156, 158, 159, 161) über Kreuz verbindet.

6. Röntgendiagnostikeinrichtung nach einem der Ansprüche 1 bis 4, d a d u r c h   g e k e n n - z e i c h n e t ,   daß die die Röntgenröhre (10) und den Filmkassettenhalter (20) verbindenden Bewegungsmittel (24) Hebel sind, die ein erstes Kniegelenk (60p), das mit seinem einen Endteil an dem ersten Hebelarm (30) und mit seinem zweiten Endteil mit einer gemeinsamen drehbaren Achse (40) verbunden ist, die an der Befestigungsvorrichtung (22) angeordnet ist, und daß die Mittel ein zweites Kniegelenk (62p) aufweisen, das mit seinem einen Ende mit dem zweiten Hebelarm (34) und mit seinem anderen Endteil mit der gemeinsamen drehbaren Achse (40) verbunden ist.

7. Röntgendiagnostikeinrichtung nach Anspruch 6, d a d u r c h   g e k e n n z e i c h n e t ,   daß eine Vielzahl von Hebeln in einer Platte (70) vereinigt sind, die eine gemeinsame Drehachse (40) aufweist, die mit drei Befestigungspunkten versehen ist, wobei einer der Punkte für das erste Kniegelenk (60p), der zweite für das zweite Kniegelenk (62p) und der dritte für die Feder (44) vorgesehen ist.

8. Röntgendiagnostikeinrichtung nach einem der Ansprüche 1 bis 7, d a d u r c h   g e k e n n z e i c h n e t, daß die die Röntgenröhre (10) und den Filmkassettenhalter (20) verbindenden Bewegungsmittel (24) zwei Systeme enthalten, die parallel zueinander, seitlich der Befestigungsvorrichtung (22) angeordnet sind.

FIG 1

FIG 2

FIG. 3

FIG 4

FIG 5